# EUROPEAN PATENT SPECIFICATION

(11) **EP 1 668 137 B1**
(45) Date of publication and mention of the grant of the patent: **19.10.2011**
(21) Application number: 04770716.1
(22) Date of filing: 27.08.2004
(51) Int. Cl.: C12N 15/82, C12N 15/11, A01H 5/00, C07K 14/47

(54) **ENHANCING ACCUMULATION OF HETEROLOGOUS POLYPEPTIDES IN PLANT SEEDS THROUGH TARGETED SUPPRESSION OF ENDOGENOUS STORAGE PROTEINS**
VERBESSERUNG DER ANREICHERUNG HETEROLOGER POLYPEPTIDE IN PFLANZENSAMEN DURCH GEZIELTE SUPPRESSION ENDOGENER SPEICHERPROTEINE
ACCUMULATION RENFORCEE DE POLYPEPTIDES HETEROLOGUES DANS DES GRAINES DE PLANTES PAR SUPPRESSION CIBLEE DE PROTEINES DE STOCKAGE ENDOGENES

(30) Priority: 27.08.2003 IS 693103; 27.08.2003 US 497923 P
(43) Date of publication of application: 14.06.2006
(73) Proprietor: ORF Liftaekni HF., 112 Reykjavik (IS)
(72) Inventor: ORVAR, Bjorn, Larus, IS-200 Kopavogur (IS)
(74) Representative: Arnason Faktor
(86) International application number: PCT/IS2004/000012
(87) International publication number: WO 2005/021765

(56) References cited:
- EP-A- 0 591 530
- EP-A- 1 312 672
- EP-A- 1 327 685
- WO-A-00/77174
- WO-A-01/35725
- WO-A-03/001902
- ONATE LUIS ET AL: "Barley BLZ2, a seed-specific bZIP protein that interacts with BLZ1 in vivo and activates transcription from the GCN4-like motif of B-hordein promoters in barley endosperm" JOURNAL OF BIOLOGICAL CHEMISTRY, AMERICAN SOCIETY OF BIOLOGICAL CHEMISTS, BALTIMORE, MD, US, vol. 274, no. 14, 2 April 1999 (1999-04-02), pages 9175-9182, XP002195304 ISSN: 0021-9258
- PEVERALI F A ET AL: "Expression of HOX homeogenes in human neuroblastoma cell culture lines." DIFFERENTIATION; RESEARCH IN BIOLOGICAL DIVERSITY. OCT 1990, vol. 45, no. 1, October 1990 (1990-10), pages 61-69, XP008044926 ISSN: 0301-4681

### SEQUENCE LISTING

<110> ORF ehf.
   ÖRVAR, Björn
<120> ENHANCING ACCUMULATION OF HETEROLOGOUS POLYPEPTIDES IN PLANT SEEDS THROUGH TARGETED SUPPRESSION OF ENDOGENOUS STORAGE PROTEINS
<130> P3505PC00
<150> IS 6931
   <151> 2003-08-27
<160> 15
<170> PatentIn version 3.1
<210> 1
   <211> 251
   <212> PRT
   <213> Homo sapiens
<400> 1
   Met Ala Met Ser Ser Phe Leu Ile Asn Ser Asn Tyr Val Asp Pro Lys
   1               5                   10                  15
   Phe Pro Pro Cys Glu Glu Tyr Ser Gln Ser Asp Tyr Leu Pro Ser Asp
               20                  25                  30
   His Ser Pro Gly Tyr Tyr Ala Gly Gly Gln Arg Arg Glu Ser Ser Phe
           35                  40                  45
   Gln Pro Glu Ala Gly Phe Gly Arg Arg Ala Ala Cys Thr Val Gln Arg
       50                  55                  60
   Tyr Ala Ala Cys Arg Asp Pro Gly Pro Pro Pro Pro Pro Pro Pro Pro
   65                  70                  75                  80
   Pro Pro Pro Pro Pro Pro Pro Gly Leu Ser Pro Arg Ala Pro Ala Pro
                   85                  90                  95
   Pro Pro Ala Gly Ala Leu Leu Pro Glu Pro Gly Gln Arg Cys Glu Ala
               100                 105                 110
   Val Ser Ser Ser Pro Pro Pro Pro Pro Cys Ala Gln Asn Pro Leu His
           115                 120                 125
   Pro Ser Pro Ser His Ser Ala Cys Lys Glu Pro Val Val Tyr Pro Trp
       130                 135                 140
   Met Arg Lys Val His Val Ser Thr Val Asn Pro Asn Tyr Ala Gly Gly
   145                 150                 155                 160
   Glu Pro Lys Arg Ser Arg Thr Ala Tyr Thr Arg Gln Gln Val Leu Glu
                   165                 170                 175
   Leu Glu Lys Glu Phe His Tyr Asn Arg Tyr Leu Thr Arg Arg Arg Arg
               180                 185                 190
   Val Glu Ile Ala His Ala Leu Cys Leu Ser Glu Arg Gln Ile Lys Ile
           195                 200                 205
   Trp Phe Gln Asn Arg Arg Met Lys Trp Lys Lys Asp His Lys Leu Pro
       210                 215                 220
   Asn Thr Lys Ile Arg Ser Gly Gly Ala Ala Gly Ser Ala Gly Gly Pro
   225                 230                 235                 240
   Pro Gly Arg Pro Asn Gly Gly Pro Arg Ala Leu
                   245                 250
<210> 2
   <211> 1173
   <212> DNA
   <213> Hordeum vulgare
<400> 2
   atggagcgcg tcttctccgt cgaggagatc cccgacccgt tctggggcca gccgtcgccg 60
   cggcagcgcg ggcgccggcc gccggagggc gccatgaacc ggtgcccgtc cgagtggtac 120
   ttccagaagt tcctcgagga ggccgtgctc gacagccccg ccgccgaccc tagcccgatg 180
   tccggtgcta gcggacgagg tcaagcggcc tgccgtccgc gtggcgtggc ggggacggcg 240
   acgggcccgg cggtggaccc ggtggagtac aacgccatgc tcaagcagaa gctcgagaag 300
   gacctcgccg ccgtcgccat gtggagggcc tctggtgcca tgcctccaga acgttttgca 360
   gctagtccgt catgcccaaa tgcagatggt cagcatatag gcactattaa tcccatagga 420
   ggtaatgtgg ttccacttca aaacaagcta gctggtggcg caagcggggt gtcgggtcca 480
   catctggtac aaaatgctga tgcccttgta aagcaagccg ctagctcttc ttcgcgggag 540
   cagtcagaag atgatgatat ggaaggagaa gatgagatca ctgggaatgg ggtccctact 600
   gatcaaaggc tgcggaggag gaagcaatcc aatcgggaat cggccaggcg ttcaagaagc 660
   agaaaggcag ctcacctgaa tgaactcgaa gcacaggtat cacagttaag agttgaaaac 720
   tcctcgctgt taaggcggct tgctgatgtt aatcagaaat acaatggtgc tgctgttgac 780
   aatagggtgt taaaggcgga tgttgaaacc ttaagagcaa aggtgaagat ggccgaggac 840
   tcggtgaagc gggtgacagg catgagcgct ctgttccctg cagggtccga catgtcatct 900
   ctcagcatgc ccttcactgg ctctccatca gaagccacct ccgacgctgc gttcccggac 960
   gacctgagcg cttacttctc cacaagcgag gctggaggta acaacggata catgcccgag 1020
   atggcttcct cggcgcaaga ggatgacaac ttcctcaacg agaccatgga taccagcaag 1080
   atgggcagac ccgactcgct gcatcgtgtg gcgagcctgg agcacctcca gaagaggatg 1140
   tgcggcgggc cagcttcgtc cggatcgacc tca 1173
<210> 3
   <211> 334
   <212> DNA
   <213> Hordeum vulgare
<400> 3
   ggccgaggac tcggtgaagc gggtgacagg catgagcgct ctgttccctg cagggtccga 60
   catgtcatct ctcagcatgc ccttcactgg ctctccatca gaagccacct ccgacgctgc 120
   gttcccggac gacctgagcg cttacttctc cacaagcgag gctggaggta acaacggata 180
   catgcccgag atggcttcct cggcgcaaga ggatgacaac ttcctcaacg agaccatgga 240
   taccagcaag atgggcagac ccgactcgct gcatcgtgtg gcgagcctgg agcacctcca 300
   gaagaggatg tgcggcgggc cagcttcgtc cgga 334
<210> 4
   <211> 1230
   <212> DNA
   <213> Hordeum vulgare
<400> 4
   atggagcccg tgttctcact gctggaggag gcgatgcccg agcccgactc taaccccggt 60
   cggacctcgc cgccgcagct gcaggcacac gtgctcgccg gaggagtcag aggagcagga 120
   ggagtgggcg tcggtgagat cgttggcgat ggcgcgacag aattgtgctt cgacaagtcc 180
   atggaggagc cgtcgctgct caacgtcccc acggagccag tggcgaaccc ggacgcctcg 240
   acgcttcacc ctaatcccac ggcggaggtg agccgcaaga ggcggtacga cgttcatgag 300
   gaggaggagg tggtgggggt catccccacg ccgcctgcgg cgggcgcggt gctggacccc 360
   gtgggctaca acgcgatgct gagacggaag ttggacgcgc atctcgccgc cgtcgccatg 420
   tggaggacta ctcgaggaat ttgccgacaa agctcccatg acaatagagc atcacaaaat 480
   ccagattcta tccaaggctc agaaaatcac actggagatg ctagtgtgca acaacttagc 540
   tcttcctcat gggagccatc accatcggat gatgatatgg aaggggaggc acaaacaatt 600
   ggaactatga atattagtgc agagaaagtg aacaaaagaa aagaatctaa ccgggattcg 660
   gcgagacgct caaggagtag aaaagcagct catacgaagg aactagagga gcaagtctca 720
   ctattaagag ttgcaaataa ctccttgatg agacatctag cagatgtaag tcacagatac 780
   gtcaataccg ctattgacaa tagggtattg aaggcaaatg ttgaaaccct agaagcaaag 840
   gtaaagatgg ccgaggaaac tatgaagaga attacatcca ccaacaattt cccccaagca 900
   atatctggca tgtcatctct caggacccat ttcagtggtt cccaattgga tggcatcttt 960
   gatactacat tgccaaccca aaacatgtca cttaaccatt tttccactac agcaacaaat 1020
   tttgatgtga gcagcaacta catccccgag ctagctccgg cataccagat ccatgatcaa 1080
   atatcttcgc tacatacgca acctatgcca tgcttggatc atcacccacg gaggatgccc 1140
   tttggtattc caagtacatt ggtgcctacc ccacaacggg aatccactac attggattca 1200
   aatgaaatag gcaacatggt gatgcagtag 1230
<210> 5
   <211> 733
   <212> DNA
   <213> Hordeum vulgare
<400> 5
   gatactacat tgccaaccca aaacatgtca cttaaccatt tttccactac agcaacaaat 60
   tttgatgtga gcagcaacta catccccgag ctagctccgg cataccagat ccatgatcaa 120
   atatcttcgc tacatacgca acctatgcct gcttggatca tcacccacgg aggatgccct 180
   ttggtattcc aagtacattg gtgcctaccc cacaacggga atccactaca ttggattcaa 240
   atgaaatagg caacatggtg atgcagtagg aattatgtga gagacctgag ccggaattat 300
   tattttaaaa taaatattgt cgttgttctt ggtggccatt gggggtattt gtaatggttt 360
   ctcaccttgt taagctcaga cttgtttaag gccggccggg gccgaggact cggtgaagcg 420
   ggtgacaggc atgagcgctc tgttccctgc agggtccgac atgtcatctc tcagcatgcc 480
   cttcactggc tctccatcag aagccacctc cgacgctgcg ttcccggacg acctgagcgc 540
   ttacttctcc acaagcgagg ctggaggtaa caacggatac atgcccgaga tggcttcctc 600
   ggcgcaagag gatgacaact tcctcaacga gaccatggat accagcaaga tgggcagacc 660
   cgactcgctg catcgtgtgg cgagcctgga gcacctccag aagaggatgt gcggcgggcc 720
   agcttcgtcc gga 733
<210> 6
   <211> 346
   <212> DNA
   <213> Hordeum vulgare
<400> 6
   ccgcggactg ggaatggggt ccctactgat caaaggctgc ggaggaggaa gcaatccaat 60
   cgggaatcgg ccaggcgttc aagaagcaga aaggcagctc acctgaatga actcgaagca 120
   ctcgagcagg tttgacaatt tacattgttg tttcttgact ggcagtggat tctgaaaaag 180
   gcgctaatga gtttttgttg tggatggctc gttacaggtt agatcttgct tcgagttcat 240
   tcaggtgagc tgcctttctg cttcttgaac gcctggccga ttcccgattg gattgcttcc 300
   tcctccgcag cctttgatca gtagggaccc cattcccagt tctaga 346
<210> 7
   <211> 358
   <212> DNA
   <213> Artificial
<220>
   <223> synthesized portion of BLZ1 cDNA
<400> 7
   ccgcggacaa acaattggaa ctatgaatat tagtgcagag aaagtgaaca aaagaaaaga 60
   atctaaccgg gattcggcga gacgctcaag gagtagaaaa gcagctcata cgaaggaact 120
   agaggactcg agcaggtttg acaatttaca ttgttgtttc ttgactggca gtggattctg 180
   aaaaaggcgc taatgagttt ttgttgtgga tggctcgtta caggttagat cttcctctag 240
   ttccttcgta tgagctgctt ttctactcct tgagcgtctc gccgaatccc ggttagattc 300
   ttttcttttg ttcactttct ctgcactaat attcatagtt ccaattgttt gttctaga 358
<210> 8
   <211> 857
   <212> DNA
   <213> Artificial
<220>
   <223> sequence encoding hpRNA, stem from both BLZ1 andBLZ2
<400> 8
   aagcttccgc ggactgggaa tggggtccct actgatcaaa ggctgcggag gaggaagcaa 60
   tccaatcggg aatcggccag gcgttcaaga agcagaaagg cagctcacct gaatgaactc 120
   gaagcaacaa acaattggaa ctatgaatat tagtgcagag aaagtgaaca aaagaaaaga 180
   atctaaccgg gattcggcga gacgctcaag gagtagaaaa gcagctcata cgaaggaact 240
   agaggactcg agcaggtttg acaatttaca ttgttgtttc ttgactggca gtggattctg 300
   aaaaaggcgc taatgagttt ttgttgtgga tggctcgtta caggttagat cttcctctag 360
   ttccttcgta tgagctgctt ttctactcct tgagcgtctc gccgaatccc ggttagattc 420
   ttttcttttg ttcactttct ctgcactaat attcatagtt ccaattgttt gttgcttcga 480
   gttcattcag gtgagctgcc tttctgcttc ttgaacgcct ggccgattcc cgattggatt 540
   gcttcctcct ccgcagcctt tgatcagtag ggaccccatt cccagttcta gagaagcaga 600
   tcgttcaaac atttggcaat aaagtttctt aagattgaat cctgttgccg gtcttgcgat 660
   gattatcata taatttctgt tgaattacgt taagcatgta ataattaaca tgtaatgcat 720
   gacgttattt atgagatggg tttttatgat tagagtcccg caattataca tttaatacgc 780
   gatagaaaac aaaatatagc gcgcaaacta ggataaatta tcgcgcgcgg tgtcatctat 840
   gttactagat cgaattc 857
<210> 9
   <211> 39
   <212> DNA
   <213> Artificial
<220>
   <223> synthesized primer
<400> 9
   ggaattccct tcgagtgccc gccgatttgc cagcaatgg 39
<210> 10
   <211> 41
   <212> DNA
   <213> Artificial
<220>
   <223> synthesized primer
<400> 10
   ataagaatgc ggccgcaatg aattgatctc tagttttgtg g 41
<210> 11
   <211> 414
   <212> DNA
   <213> Hordeum vulgare
<400> 11
   ccttcgagtg cccgccgatt tgccagcaat ggctaacaga cacatattct gccaaaaccc     60
   cagaacaata atcacttctc gtagatgaag agaacagacc aagatacaaa cgtccacgct    120
   tcagcaaaca gtaccccaga actaggatta agccgattac gcggctttag cagaccgtcc    180
   ctgttttgca aagctccaat tcctccttgc ttatccaatt tcttttgtgt tggcaaactg    240
   cacttgtcca accgattttg ttcttcccgt gtttcttctt aggctaacta acacagccgt    300
   gcacatagcc atggtccgga atcttcacct cgtccctata aaagcccagc caatctccac    360
   aatctcatca tcaccgagaa caccgagaac cacaaaacta gagatcaatt catt          414
<210> 12
   <211> 29
   <212> DNA
   <213> Artificial
<220>
   <223> synthesized primer
<400> 12
   ggaattccat ggctaagcgg ctggtcctc 29
<210> 13
   <211> 30
   <212> DNA
   <213> Artificial
<220>
   <223> synthesized primer
<400> 13
   ggaattcctt gcaattggat aggtctcttg 30
<210> 14
   <211> 3
   <212> DNA
   <213> Hordeum vulgare
<400> 14
   ggg 3
<210> 15
   <211> 791
   <212> DNA
   <213> Artificial
<220>
   <223> synthesized cDNA
<400> 15

## Description

### FIELD OF THE INVENTION

The present invention is in the field of plant molecular biology and relates specifically to methods to increase expression of heterologous proteins in plant seeds by suppressing competition from endogeneous expression of seed storage proteins in endosperm of monocotyledonous plants.

### BACKGROUND

Many plants express and store in the endosperm of developing seeds a large reservoir of proteins that serve different functions, such as different kinds of storage proteins, metabolic enzymes, endochitinases, protein synthesis inhibitors, protease inhibitors, amylases, lectins and peroxidases. The storage proteins in endosperm can amount to more than 15% of seed dry weight, such as is common in many monocotyledonous crop species. Accordingly, at certain developmental stage, the cell machinery in seed endosperm is primarily occupied with production and storage of these particular proteins. Furthermore, most storage protein genes are found in multiple copies, reflecting the importance of fast accumulation of these proteins, in the span of a few days to a few weeks.

The inherent biological capacity for protein accumulation in developing endosperm of crop seeds means that many crop plants, especially monocotyledonous, have the potential to be a practical and efficient vehicle for large-scale production of heterologous recombinant proteins, e.g. high-value polypeptides for the pharmaceutical industry; a manufacturing process often referred to as molecular farming. In addition, storing heterologous polypeptides in seeds reduces down-stream processing cost since these seeds may be stored for years without affecting the quality of the heterologous polypeptide. Expression of such proteins are, preferably, under the control of seed-specific or endosperm-specific promoters.

General molecular biology strategies, such as selection of appropriate promoters and sub-cellular localization, are utilized when improving the expression levels of the heterologous protein in the particular plant used for molecular farming, although the expression level is also critically affected by the nature of the protein to be expressed, its complexity and function. However, low expression levels of the heterologous protein (low percentage total soluble protein or %TSP) has been a particular concern, even when expressed in seeds. A novel biotechnological approach is required to enhance further the expression level. This is a considerable challenge, especially since the cellular mechanism is already programmed for its endogeneous role which, in developing seeds, is preferably occupied with accummulation of storage proteins and general preparation for dormancy-dependent survival. The promoters most frequently used for driving the expression of the heterologous gene of interest in endosperm or seeds of monocotyledonousplants are, largely, promoters from storage protein genes, such as the GluB-1, a 1.3 kb endosperm-specific promoter from rice (Patel et al. 2000; GenBank Accession No. X54314), or the 0.45 kb D-hordein promoter from barley (Sörensen et al. 1996; GenBank Accession No. X84368). Although these promoters are strong for driving expression of heterologous proteins of interest, their activity in time and space coincide with activity of endogeneous promoters driving the most abundant storage proteins, causing competition over limited resources, such as amino acids, ribosomal binding sites and the ensemble of enzymes participating in translation and post-translational processing. This competition negatively affects the expression levels of the heterologous polypeptide of interest.

It would be highly desirable to have a method to suppress the expression of endogeneous genes, such as many of the endogeneous storage proteins genes that are actively competing for resources, in time and space, with the expression of the heterologous protein of interest. Therefore, suppression of storage proteins could have various industrial needs if a practical method of suppression could be provided. This can be a challenge of a considerable magnitude in molecular farming, especially if the endogeneous gene is a member of a multigene family. In barley, for example, the major sink for resources in endosperm are the B-hordein storage proteins that may account for as much as 50% of total endosperm proteins (Shewry 1993: in Barley; Chemistry and Technology).

The term "sink", "sink genes" and "sink proteins" refer herein to genes and gene products that are actively expressed and take up a substantial amount of available resources in the cell, i.e., a sink "drains" a cell of a substantial portion of its resources, and therefore limits available resources for the expression of other genes and gene products.

Antisense technology is emerging as an effective means for reducing the expression of specific endogenous gene products in plant cells (see, e.g., US patent No. 5,759,829; Örvar et al. 1997; Coles et al. 1999). However, this technology is not a practical approach to suppress directly expression of genes that are members of a gene family, which is frequently the case with seed storage proteins, such as the B-hordein genes that are at least 20 per haploid genome (Shewry et al. 1985); the technology is better suited to manipulate expression of genes not belonging to a gene family.

Another method for reducing endogeneous gene expression is the application of double-stranded RNA (dsRNA) for post-transcriptional gene silencing (PTGS). This RNA-induced gene silencing or RNA-interferance, where both the sense and its complementary antisense RNA strand of a particular endogeneous gene are combined in one dsRNA, was first shown in Caenorhabditis elegans (Fire et al. 1998). Hamilton et al. (1999) indicate that short nucleotide RNAs (21-23 nucleotides long) are break-down products of the dsRNA that regulate the clevage of the endogeneous target mRNA in PTGS in plants. A more powerfull approach of double-stranded gene silencing in plants has been the method described by Wesley et al (2001) where a gene silencing gene is constructed in such a way that it forms a "hairpin-loop" RNA (hpRNA) following transcription. One form of the hpRNA gene silencing is the intron-spliced hpRNA (ihpRNA) where the spacer in the hairpin-loop is an intron. The ihpRNA is capable of very strong PTGS in plants (see also Waterhouse et al 2001 and Smith et al 2000). Methods for producing such PTGS constructs in a special vector are described in US Patent application 2003-A-0049835.

EP 1312672 discloses a transformed plant cell defective in endogenous seed storage protein into which a foreign gene has been introduced by cross-fertilization. The disclosure makes use of mutants that are defective and therefore do not express a major seed storage protein, and suggests, as an alternative to such mutants, to generate plants with reduced level of seed storage protein by co-suppression or the antisense method, i.e. enabling direct suppression of the gene encoding the storage protein to be reduced.

EP 1327685 discloses the identification and provision of a RISBZ1 transcription factor In rice, and the use of said transcription factor to enhance the expression of seed storage proteins or a desired recombinant foreign protein encoded by a foreign gene introduced in a transformed plant cell, downstream of a promoter containing the target sequence of RISBZ1.

There is a need for new methods for improving the expression of heterologous genes in molecular farming and especially in monocotyledonous plants such as barley. The ability to reduce competition in expression between heterologous genes of interest and endogeneous "sink" genes for the purpose of increasing expression of pharmaceutical proteins of interest in molecular farming, has not been accomplished in the art. The prior art has also not demonstrated how targeted gene silencing, using for example hpRNA-induced PTGS, can in fact increase expression levels of heterologous genes of interest in molecular farming.

### SUMMARY AND OBJECTS OF THE INVENTION

The primary objective of present invention is summarized as providing methods and nucleic acid constructs for increasing levels of accummulation of heterologous polypeptides of interest in transgenic seeds used as a production vehicle in molecular farming. A primary approach is to limit competition for resources from protein translation of endogeneous undesired mRNA encoding endosperm-specific storage proteins in monocotyledonous plants in favour of expression of the recombinantly produced heterologous polypeptide of interest in aforementioned endosperm.

Previous methods to suppress expression of genes that are members of a gene family is to target an individual member or homologous regions within the coding sequence of gene family members that are characteristic to all of them. One objective of the present invention is to avoid the disadvantages of these previously described methods, by attenuating the expression of the gene family by suppressing expression of single-gene transcription regulators that in a coordinated fashion orchestrate regulation of the expression of a plurality of members of the aforementioned gene family.

Another objective of the present invention is to use hpRNA-induced PTGS of transcription regulators to suppress the expression of major storage proteins in the endosperm of monocotylodonous plants, and, thereby, avoid using so called antisense technology which is more conventional in the art, or the so called co-suppression, for the same purpose.

If hpRNA-induced PTGS of transcription regulators of major storage protein genes, such as the hordeins in barley, could be achieved, without affecting expression levels of transcription regulators of the particular promoter driving the transgene coding for the heterologous protein of interest, competition for limited resources for translation would be reduced in favour of the mRNA encoding the heterologous protein of interest, leading to increased accumulation of the particular heterologous protein of interest.

An objective of the present invention is to provide methods for suppressing expression of transcription regulators of major storage protein genes, and, thus, reducing the expression levels of said storage protein genes, without affecting activity of the particular promoter driving the expression of the transgene of interest, encoding the heterologous protein being produced in the plant.

A first aspect of the invention provides a method of enhancing the expression and accumulation of a heterologous polypeptide of interest in plant seeds, said method comprising:
(a) transforming a plant cell with a DNA sequence for a seed-specific promoter operably linked to a DNA sequence encoding one or more transcription regulators (TF), or part(s) thereof, including a chimeric combination of different TF(s), regulating transcription of one or more endogenous genes encoding seed storage proteins, wherein the transcribed strand of said TF DNA sequence is capable of forming a "hairpin" RNA capable of suppressing, delaying or otherwise reducing the expression of one or more of said seed storage proteins in said plant cell, and
(b) selecting a seed-specific promoter that has no cis-acting elements recognized by the transcription regulators described above in (a), and
(c) transforming the same or another plant cell described in (a) with a DNA sequence for a promoter described in (b) operably linked to a DNA sequence encoding a heterologous polypeptide of interest;
(d) regenerating a plant from said transformed plant host cell(s), and growing said plant under conditions whereby said DNA sequence(s) encoding one or more TF(s), or part(s) thereof, is transcribed, thereby reducing expression of said endogenous mRNA, thus reducing expression of said seed storage proteins, and thus enhancing the expression and accumulation of said heterologous polypeptide of interest.

In a useful embodiment, the DNA sequence(s) of step (a) and the DNA sequence of step (c) are introduced into the same plant cell. In such embodiments, the sequence may be operably linked in one DNA sequence.

However, in other interesting embodiments, the DNA sequence(s) of step (a) is introduced into the genome of a first plant host cell, and the said DNA sequence of step (c) is introduced into the genome of a second plant host cell. A first transgenic plant is then regenerated from said first plant host cell and a second transgenic plant is generated from said second plant host cell, and a progeny population of transgenic plants is generated from sexual crossing between said first and second transgenic plant, the progeny population plants having cells comprising both the the DNA sequence(s) encoding said one or more TF-s, and the DNA-sequences encoding the heterologous protein of interest, such that the plants are able to express and accumulate said heterologous protein.
In preferred embodiments, the suppressed seed storage protein is a hordein of barley, e.g., one or both of B-hordein and C- hordein.

The DNA sequence encoding the heterologous polypeptide of interest can be DNA sequence encoding a prokaryotic or eukaryotic protein, that may be accumulated in a plant cell according to the invention. Examples of proteins that may selected for production according to the present invention are collagens, collagenase, homeobox polypeptides, monoclonal antibodies, secreted antibodies, single chain antibodies, mannose-binding lectin, pepsin, chymotrypsin, trypsin, casein, human growth hormone, human serum albumin, human insulin, lactoferrin, lyzosymes, cellulases, pectinases, hemicellulases, phytases, hydrolases, peroxidases, fibrinogen, factor IX, factor XIII, thrombin, protein C, xylanase, isoamylase, glucoamylase, amylases, lysozyme, beta.-glucanase, glucocerebrosidase, caseins, lactase, urease, glucose isomerase, invertase, streptavidin, esterases, alkaline phosphatase, protease inhibitors, proteases, papain, kinases, phosphatases, deoxyribonucleases, ribonucleases, phosphlipases, lipases, laccase, spider silk proteins, antifreeze proteins, antimicrobial peptides or defensins, growth factors and cytokinins.

In some embodiments, said DNA sequence encodes a desired protein from a thermophilic organism, such as e.g. a carbohydrade binding module (CBM). An example of a suitable CBM is the CBM9-2 from *Thermotoga maritima.* The CBM9-2 genomic DNA sequence is available as GenBank Accession No. Z46264 and it belongs to the Family IX of CBM-s. Also fusion proteins comprising said CBM or another suitable CBM may be encoded by the DNA sequence and overexpressed in seeds in accordance with the invention. Methods of purifying such CBMs and CBM fusion proteins are described in further detail in applicant's copending international patent applications "A non-denaturing process to purify recombinant proteins from plants" and "A process for proteolytic cleavage and purification of recombinant proteins" filed simultaneously with this application**.**

In a particular embodiment, said DNA sequence comprises a human homeobox B4 (HoxB4) gene encoding HoxB4 protein. Said human HoxB4 protein preferably has the sequence depicted as SEQ ID NO: 1, or substantial sequence identity to SEQ ID NO: 1, such that the expressed protein preferably has all functional characteristics of the native HoxB4 protein. Substantial sequence identity indicates in the context herein at least 50% sequence identity and more preferably at least 60% such at least 70% sequence identity, such as at least 80% and preferably, at least 90% sequence identity, such as at least 95% or 99% sequence identity.

As indicated herein, said DNA sequence encoding one or more TF or part(s) thereof is preferably capable of forming a "hairpin" RNA capable of suppressing, delaying or otherwise reducing the expression of one or more of said seed storage proteins. Said DNA sequence may comprise a complete TF sequence, however, in some cases, suppression will be effected even if only a part of the TF sequence is In place. Hence, in these embodiments, a sufficiently long part of the one or more TF sequences is required such that suppression is affected. In some cases, a part of the TF sequence as short as 20 nucleotides in length is sufficient, but preferably a part in the range of at least 20-500 nucleotides is used, including about 20-200, such as in the range of 20-100, or in the range of 50-100 nucleotides in length.

In certain useful embodiments, the DNA sequence encoding one or more TF is a chimeric DNA sequence, as defined herein, comprised of regions of two or more DNA sequences encoding TF-s, or parts thereof. In other useful embodiments, the DNA sequence encoding one or more TF in a chimeric DNA sequence, may also include an intron sequence capable of forming a loop in a "hairpin" RNA.

Preferred embodiments make use of DNA sequences encoding a TF or part thereof comprises a region encoding a TF or part thereof from the group of bZIP proteins, more preferably bZIP proteins selected from barley BLZ1 and BLZ2 proteins (see Onate et al 1999). Said DNA sequence may comprise a part of the sequence encoding such protein, of sufficient length to affect suppression, or a combination of parts of the sequences encoding said proteins.

Said DNA sequence preferably comprises the sequence set forth as SEQ ID NO: 2, SEQ ID NO:3 or SEQ ID NO: 4, or a sequence encoding a protein with substantial sequence identity to any of the amino acid sequences encoded by said sequences, or a sequence with a part of SEQ ID NO: 2, or SEQ ID NO: 4, of sufficient length to cause suppression according to the method of the invention. As indicated above, any combination of said sequences, such as a combination of one or more parts of the above sequences may be useful, an example of such combination is shown as SEQ ID NO: 5 which is a chimera of parts of BLZ1 and BLZ2.

Another objective of the present invention is to provide methods to use hpRNA-induced PTGS technology to suppress expression of transcription regulators of major storage protein genes. Therefor, in certain embodiments, the methods of the invention apply hpRNA-induced PTGS technology to suppress the expression of endogeneous storage proteins and, therefore, increasing availability of resources, such as amino acids, ribosomal binding sites and the ensemble of enzymes participating in translation and post-translational processing, for translation of mRNA encoding heterologous polypeptide of interest. Hence, in preferred embodiments, said DNA sequence encoding one or more TF-s or part(s) or combination thereof, preferably from the group of bZIP proteins as described above, is capable of expressing a "hairpin" RNA (hpRNA) in said plant cell. Said DNA sequence preferably comprises a sequence selected from SEQ ID NO: 2, SEQ ID NO: 4, SEQ ID NO: 6, or SEQ ID NO: 7, SEQ ID NO: 8 and any part or combination thereof, of sufficient length to cause suppression according the methods described herein, i.e., particularly in seeds and/or endosperm, such as preferably in endosperm in barley.

Hence, an objective of the present invention is to provide methods and tools In molecular biology for reducing the levels of B-hordelns and C-hordelns In barley endosperm. More particularily, an object of the present invention is to provide gene constructs comprising regions of the BLZ1 and BLZ2 genes capable of forming hpRNA and, thus, enabling reduction of expression of said endosperm genes.

More particularily, the invention provides gene constructs of regions of the BLZ1 and BLZ2 genes encoding transcript forming a hpRNA capable of PTGS in barley endosperm.

In a preferred embodiment of the foregoing, a method of making transgenic plants with reduced level of endosperm storage proteins comprises: (1) providing a monocotyledonous plant cell, preferably barley, capable of regeneration into fertile plant; (2) transforming the plant cell with nucleic acid construct comprising an expression cassette, read in the 5' to 3' direction, a seed-specific, preferably endosperm-specific promoter, a nucleic acid sequence encoding transcription regulator regulating endogenous seed storage proteins, another seed-specific, preferably endosperm-specific promoter that has no cls-acting elements recognized by the transcription regulator regulating the storage protein gene(s) to be suppressed, a nucleic acid sequence encoding a heterologous polypeptide of interest and a 3' untranslated region; (3) regenerating the said plant cell to provide the first transgenic plant having reduced levels of transcription regulators regulating expression of seed storage proteins and having reduced levels of endogenous storage proteins.

Useful in the present invention are DNA constructs for high-level expression of heterologous polypeptides of interests in monocotyledonous seeds. Such DNA constructs include: a promoter that is functional in a given monocotyledonous seed and operably linked to a sequence encoding a heterologous polypeptide of interests; and a chimeric gene construct of regions of the BLZ1 and BLZ2 genes encoding a transcript capable of forming hpRNA capable of PTGS in barley endosperm operably linked to a promoter that is functional in a given monocotyledonous seed.

Another aspect of the invention provides transgenic barley plants, such as those described above, obtainable by the methods described herein. Said plants have the above-mentioned desired characteristics, i.e., suppress the expression of selected major resource "sink" proteins In order to enhance the expression and accumulation of desired recombinantly produced proteins. Preferred plants according to the invention include Barley plants, of the variety Hordeum vulgaris, that can be used in accordance with the invention for expression and accumulation of heterologous proteins in seeds. The Barley plants of the present invention have in their genome sequences encoding the preferred TFs described herein such as a TF or part thereof from the group of bZIP proteins.

### BRIEF DESCRIPTION OF THE DRAWINGS

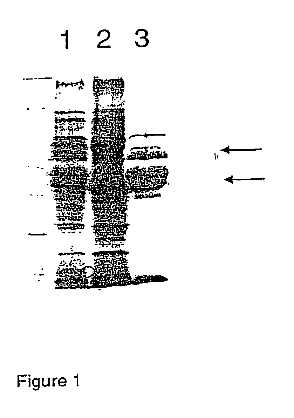

FIG. 1 Sodium dodecyl sulfate-polyacrylamide electrophoresis of total protein from Hordeum vulgaris cv. Skegia (49 days post anthesis) using aqueous extraction (lane 1), salt extraction (lane 2) and EtOH extraction (lane 3). Arrows indicate the B- (lower) and C-hordeins (upper).

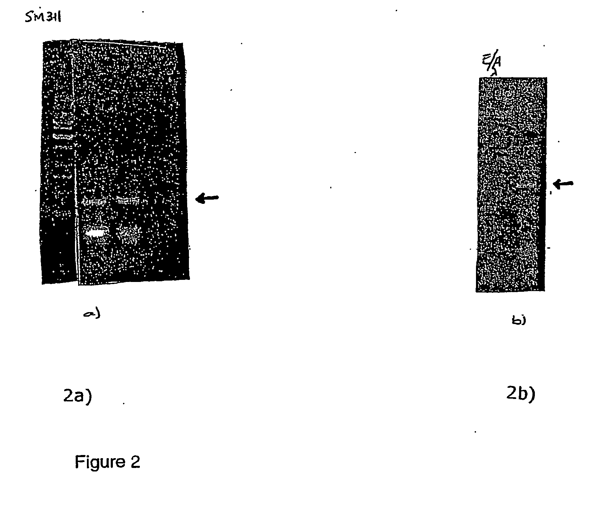

FIG. 2 Agarose gel analysis of PCR amplification products using a) primers SEQ ID NO: 9 and SEQ ID NO: 10 (see Example 2) and b) primers SEQ ID NO: 12 and SEQ ID NO: 13 (Example 3). The products were separated on 1.0% agarose gel, stained with ethidium bromide and photographed under UV. The size markers on the left are EcoRI/HindIII- cut lambda. Arrows indicate the amplified products.

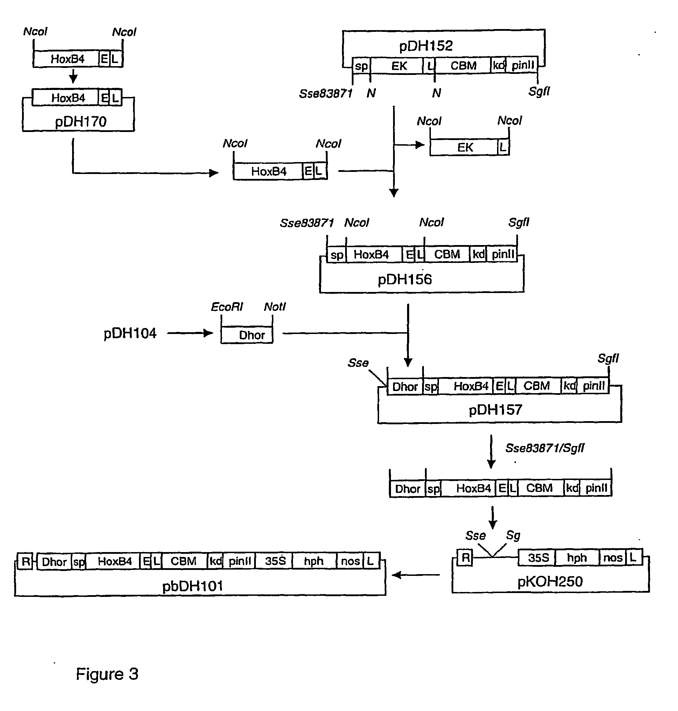

FIG. 3 illustrates schematic representation of cloning of the binary plant transformation vector pbDH101 for transformation of barley with codon-optimized HoxB4-CBM chimeric gene for expression in barley endosperm tissue under the regulation of the D-hordeln promoter. Abbreviations: D-hor, D-hordein promoter; CBM, codon-optimized gene encoding carbohydrate binding domain from Thermatoga maritima; HoxB4, codon-optimized (SEQ ID NO: 20); L, linker (PTPTPT; P is proline, T is threonine); kd, KDEL sequence; pinII, potato proteinase inhibitor II gene termination signal; CaMV 35S, cauliflower mosaic virus 35S promoter; hph, hygromycin phosphotransferase from E. coli (Genebank accession # K01193); Nos-ter, nopaline synthase termination signal; R, right border; L, left border.

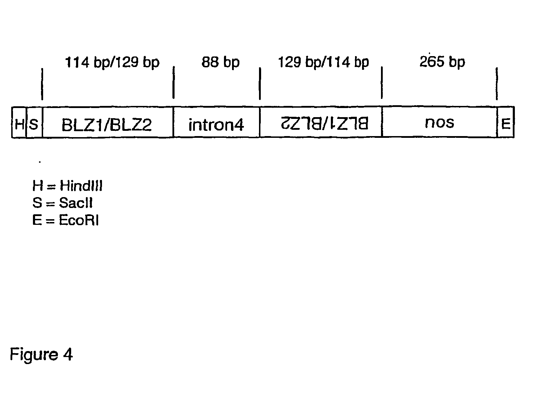

FIG. 4 is a schematic representation of the gene constructed for hairpin RNA-induced targetal suppression of BLZ1 and BLZ2 in barley under the regulation of the D-hordein promoter. The 114 bp BLZ1/BLZ2 in sense orientation and in antisense orientation, respectively, are capable of forming the stem section while the 88 bp intron4 form the loop in the hairpin loop.

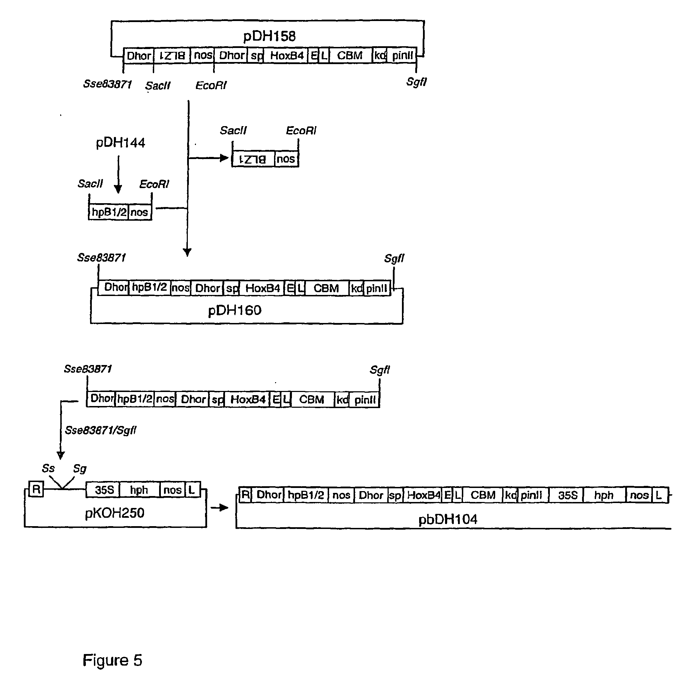

FIG. 5 illustrates schematically cloning of the binary plant transformation vector pbDH104 for transformation of barley with hpRNA-induced targetal suppression of BLZ1 and BLZ2 in barley endosperm tissue under the regulation of the D-hordein promoter. Abbreviations: D-hor, D-hordein promoter; hpB1/2, a 620 bp suppressor fragment composed of nucleotide sequences (SEQ ID NO: 8) derived from BLZ1 and BLZ2; CBM, codon-optimized gene encoding carbohydrate binding domain from Thermatoga maritima; HoxB4, codon-optimized homeo box B4 gene (SEQ ID NO: 20); L, linker (PTPTPT; P is proline, T is threonine); pinII, potato proteinase inhibitor II gene termination signal; CaMV 35S, cauliflower mosaic virus 35S promoter; hph, hygromycin phosphotransferase from E. coli (Genebank accession #K01193); Nos-ter, nopaline synthase termination signal; R, right border; L, left border.

### DETAILED DESCRIPTION OF THE PRESENT INVENTION

Herein below, the present invention will be described in detail.

The term "protein" is used herein interchangeably with "polypeptide" and "peptide".

The term "heterologous" used herein may be used interchangeably with the term "non-native" or "foreign" or "exogeneous" and applies to proteins and/or in-vitro modified DNA sequences that are normally not found in the host organisms that have not been subjected to genetic manipulation involving recombinant DNA technology. The term "heterologous polypeptide of interest" or "polypeptide of interest" used herein refers to any polypeptide intended for expression in a host organism using the methods or compositions of the present invention. As non-limiting examples, pharmacological polypeptides (e.g., for medical uses) or industrial polypeptides (e.g., enzymes) can be produced according to the present invention.

The term "coding sequence" refers to a nucleotide sequence encoding a specific amino acid sequence.

A "promoter" is defined as an array of nucleic acid control sequences or transcription regulator binding sites that direct transcription of an operably linked nucleic acid. The promoter refers to a nucleic acid sequence controlling the expression of a coding sequence or functional RNA. The term "operably linked" refers to a functional linkage between a promoter (nucleic acid expression control sequence, or array of transcription factor binding sites) and a second nucleic acid sequence, wherein the promoter directs transcription of the nucleic acid corresponding to the second sequence.

"Transcription regulators" or "Transcription factors" refers to trans-acting regulatory proteins which can bind to cis-acting elements, also called cis-acting motifs, that are short DNA sequences located upstream of genes, or within introns, or downstream of stop codon.

The term "endogenous gene" refers to a native gene in its natural location in the genome of an organism.

The term "chimeric combination" or "chimeric gene" or a "hybrid gene" refers to any two or more linked DNA sequence forming a gene that is not an endogenous gene, comprising regulatory and or coding sequences that are normally not found together in nature. The term "gene construct" or "DNA construct" or "nucleic acid construct" refers to any DNA sequences or combination of DNA sequences that are assembled by general molecular biology strategies.

The terms "expression", as used herein, refers to the biosynthesis of a gene product, including the transcription of sense (mRNA), antisense RNA or other RNA polymers thereof in either single- or double-stranded form resulting from RNA polymerase-catalyzed transcription of a DNA sequence. Expression may also refer to translation of mRNA from said gene into a polypeptide.

The term "transformation" refers to the transfer of a nucleic acid molecule into the genome of a host organism, resulting in genetically stable inheritance. Host organisms containing the transformed nucleic acid fragments are referred to as "transgenic" organisms. The term "transgenic" means that the plant host cell of the invention contains at least one foreign, preferably two foreign nucleic acid molecule(s) stably integrated in the genome. Examples of methods of plant transformation include Agrobacterium-mediated transformation (De Blaere et al. 1987) and particle-bombardment or "gene gun" transformation technology (Klein et al. (1987); U.S. Pat. No. 4,945,050).

The term "antisense inhibition" or "antisense" or "antisense suppression" refers to an antisense strand sufficiently complementary to an endogenous transcription product or mRNA such that translation and expression of the endogenous transcription product is inhibited or reduced. The term "targetal suppression" refers to using recombinant DNA technology to design and apply DNA sequences capable of encoding RNA transcripts capable of interfering and suppressing expression of endogenous genes selected for suppression in some given living organism. The term "suppressor transgene" refers to any heterologous DNA sequence encoding RNA transcripts capable of interfering and suppressing expression of endogenous genes. The term "co-suppression" refers to when a gene introduced into a cell with transformation and having identical or similar sequence as an endogenous gene in that same cell causes suppression of both the endogenous gene and the introduced gene.

"bZIP" proteins used herein, refers to regulatory proteins that contain so called basic helix/leucine zipper domains that are involved in DNA binding and dimerization and that commonly bind to DNA sequences containing 5 'ACGT'3 core.

The term "hordeins" used herein, refers to storage proteins in barley and specifically synthesized in endosperm, and are divided into 4 groups: ß- (beta, also referred as B-), D-, C- and gamma hordeins.

The term "molecular farming" used herein refers to the process of using plants to produce valuable biological compounds such as proteins for further processing

Monocotyledonous plants that can be genetically manipulated can be used in the present invention. Preferably the plant is a monocotyledonous plant, more preferably selected barley, maize, wheat, oat, and rice. Barley has many desired characteristics making it a preferred candidate for the present invention, the barley species *Hordeum vulgarisis* particularly preferred. A plant that can be genetically transformed is a plant into which a heterologous DNA sequence, including a DNA sequence for a coding region or DNA sequence encoding a RNA transcript capable of forming hpRNA, can be introduced, expressed, stably maintained, and transmitted to subsequent generations of progeny. Genetic manipulation and transformation methods have been used to produce barley plants that are using herbicides including, for instance, bialaphos or basta, or antibiotic, such as hygromycin, as selectable markers.

After selecting a suitable host plant a promoter is selected for driving the expression of the heterologous gene of interest. A number of promoters which are active in plant cells have been described in the literature. It is preferred that the promoters utilized in the DNA construct of the present invention have strong activity in tissues where the accumulation of the heterologous polypeptide of interest is desired, such as in the endosperm of seeds of monocotyledonous plants. It is further preferred that the promoter of choice is not regulated by transcription regulators that are targeted to be suppressed by the method of this invention, such as transcription regulators that regulate expression of endogenous storage protein genes that compete for resources with the expression of a heterologous gene of interest. Such promoters may be obtained from a variety of plant genetic material or from plant viruses. As described below, it is preferred that the particular promoter selected is suitable for expression of a heterologous protein in monocotyledonous seeds, more preferably in barley, and most preferably in the endosperm tissue of the seed. Cloning and analysis of such suitable promoter useful for the purpose of this invention is described in Example 2.

It is still further preferred that protein profile expression pattern in the target tissue selected for the accumulation of the heterologous protein of interest is analysed and the most abundant storage proteins identified for targetal suppression according to the invention. The analysis can rely on prior art such as data collected from published literature. This may also include monitoring simultaneously the expression patterns of thousands of genes with a micro array approach, using mRNA extracted from said tissue at time points that overlap the temporal expression profile of the promoter selected for driving the expression of the heterologous protein, or using Northern blot analysis, RT-PCR and/or Western blotting. Based on gathered information candidate genes with high expression competing in time and space with expression of the heterologous gene of interest are selected for targetal suppression according to the invention.

The recombinant plasmid of the invention can be obtained by ligating (inserting) the DNA sequences of interest into an appropriate plasmid that is replicable in a bacterial host. The DNA sequences, such as the gene encoding the heterologous protein of interest or the DNA sequences designed for targetal suppression of endogenous genes should preferably be operably incorporated into the plasmid that may contain, in addition to a promoter and for this purpose, and if desired, additional enhancer DNA sequences, scaffold-attachment regions, introns, poly(A) addition signal, ribosome binding sequence and selectable marker gene of interest such as hygromycin resistance gene, ampicillin resistance gene, bialaphos resistance gene, or the like.

### EXAMPLES

The following examples are provided to better define the present invention and to guide those of ordinary skill in the art in the practice of the present invention. Unless otherwise noted, terms are to be understood according to conventional usage by those of ordinary skill in the relevant art.

### METHODS

### RNA extraction and Northern blot analysis

Total RNA is isolated using TRIzol (Gibco BRL) according to manufacturer's instructions, and 10 µg of total RNA per lane subjected to electrophoresis, and transferred to Hybond N membrane (Amersham) by capillary action. RNA is crosslinked to the membrane using a Stratolinker (Stratagene), and the filter is hybridized with high stringency hybridization and washing as described (Davis *et al.,* 1994). Probes are radiolabeled with ³²P by random priming (T7 QuickPrime Kit®; Pharmacia) according to manufacturer's instructions. Negative digital images of EtBr-stained blots are used as loading controls.

### Barley transformation

### a) Plant material for genetic transformation

Hordeum vulgare cv Golden Promise seeds were sowed on a mixture of 75% light sphagnum peat and 25% pumice (medium grain size) and plants grown at 18 °C daytime (16 hours) and 12 °C nighttime (8 hours) and 70% relative humidity under 250 µmol m⁻² s⁻¹ of continuous light during daytime in cool-white fluorescent and subirrigated as needed with water. Under these conditions the plants grew vegetatively for about 55-95 days or until immature seeds were ready as material for transformation, which is about 8 to 14 days post anthesis. Seeds were sterilized in 3% sodium hypochlorite for 40 min in rotary shaker and rinsed with five changes of sterile water.

### b) Bacterial strains and preparation for plant transformation

Agrobacterium tumefaciens, harbouring a binary vector in trans with a Ti-plasmid possessing a vir region, is used to introduce into barley the T-DNA region with the DNA construct regulating the expression of the heterologous protein of interest. Transformation of both E. coli XL-Blue and Agrobacterium tumefaciens bacteria is done by electroporation as described by Maniatis et al., Molecular Cloning: A Laboratory Manual, Cold Spring Harbor Laboratory Press, Cold Spring Harbor, N.Y. (1982). For preparation for transformation of plant a single colony of the Agrobacterium culture is inoculated 5 ml of Agro medium (Tryptone 5 g/L, yeast extract 2.5 g/L, mannitol 5 g/L, glutamic acid 1 g/L, KH₂PO₄ 250 mg/L, MgSOₛ-7H₂O 100 mg/L, biotin 1 µg/L, pH 7.0, 25 µg/ml rifampicin, 50 µg/ml spectinomycin) and grown for 24 to 40 hours at 27°C. In sterile eppendorf tubes 200 µl of culture is added to 200 µl of 30% aqueous glycerol (previously sterilised) and culture vortexed well and left on the bench for 2 hours before storing at -80°C. For each transformation one tube is removed from the -80°C freezer, thawed and approx. 200 µl of Agrobacterium bacterial stock added to 5 ml Agro medium without antibiotic. The culture is then grown for 17 to 20 hours at 27°C before using for inoculation of plant material (see below).

### c) Preparation of barley explants for Agrobacterium-induced transformation

On day one approximately 10 barley heads were picked, approx. 8 to 14 days after anthesis, awns and seeds removed and embryos between 1.5 mm and 2 mm in size selected. The initial plant material needs to be healthy and mature, and not waterlogged, and the seeds should be green with no signs of disease or fungi. Seeds were placed in 50 ml falcon tube (no more than half full) and rinsed with 70% ethanol and ethanol then poured off. 20% bleach solution (White King) was then added and mixed for 20 minutes. In laminar airflow hood the bleach solution was poured off, the seeds rinsed with sterile water (about 5 - 8 rinses) and tube placed at 4°C O/N. On day two seeds were placed in a sterile Petri dish on the microscope platform. The position of the embryo was located, the end cut off the seed and a cut down the side of seed made. The seed was then held firmly with forceps and the middle of the seed pressured so that the embryo popped out. The embryo was held in place with forceps and scalpel blade inserted in the groove between scutellum and axis and the axis slowly excised. The embryo minus the axis was placed on a regeneration media, cut side up, in centre of Petri dish with approximately 25 embryos to a plate.
d) All binary vector where propagated in E. coli XI-Blue LB culture medium containing 100 µg/ml spectinomycin at 37°C and the vector subsequently purified from 100 ml culture grown overnight using the QIAGEN® Plasmid Midi Kit. The purified binary vector was introduced into the Agrobacterium tumefaciens with electroporation by placing 1 µl (1 µg) of the vector in sterile cuvette with 0.1 cm gap (BioRad), washing down the vector with 40 µl of electrocompetent cells, and setting the voltgage at 2.5 kV and capacitance at 21 µF for the electroporation. The cells were spread on YEP selection plates containing 100 µg/ml spectinomycin and 20 µg/ml rifampicin grown at 28 °C for 2 days. Plasmid restriction digest analysis from A. tumefaciens transformants where then carried out to verify the intactness of the binary vector.

### e) Agrobacterium infection of barley explants

Approximately 20 µl of Agrobacterium culture was pipetted onto each embryo ensuring all embryos came in contact with the solution. The embryos were flipped (cut side down) and dragged across the regeneration media to the outside of plate, removing excess Agrobacterium. The embryos were then transferred to fresh regeneration media plates (cut side up) at evenly spaced intervals (25 per plate) and placed in dark cabinet at 24°C f) Regeneration and organogenesis in barley tissue culture after Agrobacterium infection After three days the embryos were transferred to a fresh regeneration media with a selectable marker, such as bialaphos or hygromycin and left there for four to six weeks, subculturing every two weeks. To regenerate shoots, the calli are transferred to shoot-induction media (SIM) and surviving callus and regenerating shoots transferred to fresh SIM every two weeks until small plantlets are formed. Then the plantlets are transferred to root-induction media (RIM) and surviving plants potted in soil for further screening.

### DNA extraction, PCR and cloning

Genomic DNA for PCR amplification was extracted from ca 200 mg young leaf tissue using the NucleoSpin Plant Kit® from Clonetech, and according to manufacturer's instructions. The PCR reactions were carried out in a peltier thermal cycler (MJ Research, PTC-200) with heated lid. All primers were synthesized chemically with a fully automated DNA synthesizer (Perkin-Elmer). All DNA fragments were size-fractionated on 1% EtBr-stained agrose gel under UV light and purified using the QIAquick® Gel Extration Kit (Qiagen). Restriction enzymes were purchased from New England Biolabs Inc (Beverly, Mass.) and Fermentas and used according to the manufacturer's instructions. Cloning and other DNA manipulations were carried out according to standard procedures as described by Maniatis et al., Molecular Cloning: A Laboratory Manual, Cold Spring Harbor Laboratory Press, Cold Spring Harbor, N.Y. (1982) but all ligation reactions used the following conditions: T4 DNA ligase (2.5U) in a buffer [50 mM Tris-HCl (pH 7.6), 5 mM MgCl₂, 1 mM DTT, 0.5 mM ATP, 2.5% polyethylene glycol-8000] at 16°C for 16 hours. Competent Escherichia coli XL-Blue were used as recipient in transformation experiments for DNA manipulations and construction of plasmid clones. All plasmid DNA midi prep were prepared using the Qiagen® Plasmid Midi Kit (Qiagen) and sequencing of DNA fragments carried out using automated DNA sequencing (ABI, Pharmacia).

### EXAMPLE 1

### Analysis of "sink" proteins in Hordeum vulgaris endosperm

Protein expression analysis in Hordeum vulgaris seeds cultivar Skegla may be carried out using SDS-PAGE of proteins extracted from seeds collected at 49-days post anthesis (dpa) to identify those endosperm-expressed proteins that are major contributors to the total protein level of the endosperm and, therefore, potential candidate for targetal suppression. Whole seeds from five axes of barley were finely ground by hand in liquid nitrogen before addition of the appropriate extraction buffer. Two kinds of extraction buffers, in addition to aqueous extraction, where used on both samples. Firstly, the extraction of total proteins was performed under reducing conditions with ethanol extraction (70% EtOH) in the presence of 1 % 2-Mercaptoethanol, 10 mM Tris-HCl pH 8.0 and 1% Polyvinyl pyrrolidine (MW 360.000). Secondly, the extraction of total proteins was performed under reducing conditions in the presence of 170 mM NaCl, 1 % 2-Mercaptoethanol, 10 mM Tris-HCl pH 8.0 and 1% Polyvinyl pyrrolidine (MW 360.000). After grinding the sample in liquid nitrogen 5 ml of the extraction buffer was added to the extraction vial followed by continous grinding for 3 min. The extract was clarified by centrifugation at 4000 rpm for 10 min at 4°C, followed by a tenfold centrifugal concentration in Ultrafree-4 concentrators with molecular 5 kDa cut-off (UFV4BCC00-Millipore Corp. Bedford, MA, USA). A 100 µl sample of the clarified, concentrated extract was added to 100 µl of 2x sample buffer and the mixture placed in a boiling water bath for 5 min. After cooling, 10 µl of the sample was loaded on 12 % polyacrylamide gel separated with SDS-PAGE. After SDS-PAGE the gel was stained with Coomassie blue R250 staining, and destained to visualize the protein bands. Results show that the most abundant proteins in developing endosperm in H.vulgare cultivar Skegla are B- and C-hordeins (Fig.1).
The results indicate that suppressing transcription regulators that regulate the genes encoding B and C hordeins is very likely to reduce competition for resources and allow increased accumulation of an expressed heterologous polypeptide, in accordance with the invention.

### EXAMPLE 2

Selection of promoter for targetal suppression of B- and C-hordeins in Hordeum vulgare used as an expression vehicle for heterologous proteins

The gene promoters of B-hordeins (GenBank Accession No. X53690) and C-hordeins (GenBank Accession No. M36941) contain 100% identical cis-acting elements identified in both of them that participate in their transcriptional regulation (Muller and Knudsen 1993; Vicente-Carbajosa et al. 1992). These cis-elements are within the so-called endosperm box (EM) located about 300 bp upstream of the translational start codon. EM harbours two distinctive cis-acting motifs for transcription factor binding, the prolamin box (PB), 5'-TGTAAAG-3', and the GCN4-like motif (GLM), 5'-(G/A)TGA(G/C)TCAT-3'. The GLM is recognized by two transcription factors, BLZ1 (GenBank Accession No. X80068), and BLZ2 (GenBank Accession No. X80068). Upon binding, transcription of the particular gene is activated (see Onate et al. 1999). Both these genes are single copy genes and therefore good candidates for targetal suppression described by this invention. Vicente-Carbajosa et al. (1998) tested their antisense suppression in transient expression system without investigating the effect on plant stably transformed plants, or on storage protein accumulation. Furthermore, the effect of such suppression on accumulation of heterologous proteins in the same tissue has not been investigated. An important criteria in engineering targetal suppression according to the invention is that the promoter driving the transgene encoding the heterologous protein of interest should not contain cis-acting motifs recognized by the particular transcription regulator responsible for expression of the storage proteins to suppress. As the B- and C-hordeins contain the GLM recognized by BLZ1 and BLZ2 the B- and C-hordein promoters cannot be used to drive the expression of the heterologous transgene encoding the desired heterologous protein. Another important criteria is that the promoter driving the suppressor transgene itself is driven by a tissue-specific, e.g. endosperm-specific, promoter so the expression of the transcription regulators in other tissues, where they may be needed, would not be affected. This is important since many transcription factors, such as the BLZ1 in barley, are expressed in more than one tissue. Still another criteria is to avoid "auto-suppression" of the suppressor transgene by using a promoter to drive the suppressor transgene that does not have cis-acting elements recognized by transcription regulators to be suppressed by the invention.

Based on these criteria, a candidate promoter was selected for targetal suppression of Band C-hordeins.

Isolation, cloning and analysis of D-hordein promoter from Hordeum vulgare cv Skegla

Primers were designed to amplify the proximal promoter region from -435 to -16 based on the translation initiation site of the D-hordein gene as described in GenBank sequence id. X84368 as a sense primer,
5'GGAATTCC_{EcoRI}CTTCGAGTGCCCGCCGATTTGCCAGCAATGG (SEQ ID NO: 9), with an EcoRI restriction site introduced at the 5'end, was synthesized and as an antisense primer,
5'ATAAGAATGCGGCCGC_{NotI}AATGAATTGATCTCTAGTTTTGTGG (SEQ ID NO: 10), with a NotI restriction site introduced at the 5'end, was synthesized. The purpose for introduction of these restriction sites at the 5'end of the primers was to aid in cloning the amplified fragment. The composition of the reaction solution used to amplify the 420 bp fragment, using the genomic DNA from Hordeum vulgare cv. Skegla as the template, was the following:

| | |
|---|---|
| Genomic DNA solution | 4 µl (50 ng) |
| Sterilized water | 12 µl |
| 10x PCR buffer [100 mM Tris-HCl (pH 8.8) | |
| 500 mM KCI, 0.8% Nonidet P40] | 2,5 µl |
| 50 pmol/µl Sense primer | 1 µl (50 pmol) |
| 50 pmo1/µl Antisense primer | 1 µl (50 pmol) |
| MgCl₂ | 1 µl (2.5 mM) |
| dNTP | 2.5 µl (1 mM) |
| Taq DNA polymerase (Fermentas) | 1 µl (5 U) |
| Total | 25 µl |

The above reaction solution was mixed thoroughly, except 9µl of sterilized water and the Taq DNA polymerase, and the solution heated to 94°C for 3 min., then cooled down to 80°C for 30 sec and the Taq DNA polymerase and 9µl of sterilized water added. The first cycle of the PCR was performed as follows: The reaction was heated to 94°C for 30 sec, cooled to 57°C for 30 sec for annealing , and then heated to 72°C or 45 sec for extension. The next 30 cycles were as follows: thermal denaturation at 94°C for 30 sec, annealing at 62°C for 30 sec and extension at 72°C for 45 sec. After completion of the 30 cycles the reaction was heated for 72°C for 4 min. The 420 bp amplified fragment (Figure 2a) was digested with EcoRI/NotI and then ligated to the EcoRI/NotI I site of vector pKOH122 to yield the recombinant plasmid pDH104. The nucleotide sequence of the 420 bp DNA insert was then decided (SEQ ID NO: 11). According to the sequence the D-hordein promoter does not contain the GLM and, therefore, suppression of B- and C-hordeins through targetal suppression of either BLZ1 or BLZ2 (see discussion above) would not affect the activity of the D-hordein promoter and, therefore, expression of the heterologous protein of interests. Furthermore, since D-hordein promoter is exclusively endosperm-specific this promoter is useful for driving suppressor transgene designed to suppress expression of BLZ1 and BLZ2.

As it is critical to avoid "auto-suppression" of the suppressor transgene, by using promoter to drive the suppressor transgene that does not have cis-acting elements recognized by transcription regulators to be suppressed by the invention, using the D-hordein promoter, that lacks the GLM, to drive suppressor gene suppressing BLZ1 and BLZ2, would not cause such "auto-suppression".

### EXAMPLE 3

Cloning of D-hordein coding region and analysis of endogenous expression of D-hordein gene at mRNA level

A sense primer, 5'GGAATTCC_{EcoRI} ATGGCTAAGCGGCTGGTCCTC (SEQ ID NO: 12), and an antisense primer, 5 'GGAATTCC_{EcoRI}TTGCAATTGGATAGGTCTCTTG (SEQ ID NO: 13), were designed to amplify a 727 bp fragment from the coding region of the D-hordein gene as described in GenBank sequence id. X84368. The composition of the reaction solution used to amplify the 727 bp fragment, using the genomic DNA from Hordeum vulgare cv. Skegla as the template, was the following:

| | |
|---|---|
| Genomic DNA solution | 4 µl (50 ng) |
| Sterilized water | 12 µl |
| 10x PCR buffer [100 mM Tris-HCl (pH 8.8) | 2,5 µl |
| 500 mM KCl, 0.8% Nonidet P40] | |
| 50 pmol/µl Sense primer | 1 µl (50 pmol) |
| 50 pmo1/µl Antisense primer | 1 µl (50 pmol) |
| MgCl₂ | 1 µl (2.5 mM) |
| dNTP | 2.5 µl (1 mM) |
| Taq DNA polymerase (Fermentas) | 1 µl (5 U) |
| Total | 25 µl |

The above reaction solution was mixed thoroughly, except 9µl of sterilized water and the Taq DNA polymerase, and the solution heated to 94°C for 3 min., then cooled down to 80°C for 30 sec and the Taq DNA polymerase and 9 µl of sterilized water added. The first cycle of the PCR was performed as follows: The reaction was heated to 94°C for 30 sec, cooled to 60°C for 30 sec for annealing , and then heated to 72°C or 45 sec for extension. The next 30 cycles were as follows: thermal denaturation at 94°C for 30 sec, annealing at 65°C for 30 sec and extension at 72°C for 45 sec. After completion of the 30 cycles the reaction was heated for 72°C for 4 min. The 727 bp amplified fragment (Fig. 2b) was digested with EcoRI and then ligated to the EcoRI site of vector pKOH122 to yield the recombinant plasmid pDH136. The sequence of the DNA insert was then decided (SEQ ID NO: 14).

The mRNA steady-state level of the D-hordein gene in endosperm 40 DAP, embryo 40 DAP, leaves, stem and root is suitably analysed using Northern blot analysis: Total RNA is isolated from 200 - 250 mg plant material from the different tissues or plant parts from Hordeum vulgare cv Skegla, separated on 1.2% agarose-formaldehyde gel and blotted onto Zeta-Probe membrane (Millipore, Bedford, Mass.) according to supplier's instructions. Blotting, hybridization and washing are performed as described (Davis et al., 1994). The hybridization is performed using a ₃₂P radiolabeled, random-primed 747bp fragment of the D-hordein gene (SEQ ID NO: 14) as a probe.

### EXAMPLE 4

### Cloning of Barley Endosperm-codon-optimized HoxB4 into Plant Transformation Vector pbDH101

Codon-optimized HoxB4 for expression under the regulation of D-hordein promoter in barley is synthezised by GeneArt GmbH (Germany), using the basic sequence information as described in GeneBank ID NM_024015 and codon-usage information for codon-optimized expression in endosperm under the regulation of the D-hordein promoter as described (see applicant's co-pending application "Methods for high level expression of polypeptides in plants using codon optimization"). The 791 bp fragment (SEQ ID NO: 20) is flanked by NcoI sites (CCATGG) for cloning and included a 27 nucleotide sequence encoding the linker-enterokinase cleavage site DDDDKPTPTPT at the 3'end. The HoxB4 fragment is ligated into NcoI/NcoI site of pKOH122 to create pDH170. The fragment is then released with NcoI and ligated into the NcoI site in pDH152, substituting the EK-L fragment in pDH152 to yield pDH156. The D-hordein promoter fragment from pDH104 is then ligated into the EcoRI/NotI site of pDH156 to yield pDH157. The D-promoter/SP/HoxB4-E-L/CBM/kd/pinII fragment is released with Sse83871/SfI and ligated into Sse83871/SfI site of pKOH250 to generate the plant transformation vector pbDH101 (Fig.3).

### EXAMPLE 5

### Construction of plant transformation vector pbDH104 with hairpin RNA-induced targetal suppression of BLZ1 and BLZ2 under regulation of D-hordein promoter

The gene for hairpin RNA-induced targetal suppression of BLZ1 and BLZ2 was synthezised by GeneArt GmbH (Germany) as a 592 bp suppressor fragment followed by a 265 bp nos-termini fragment (SEQ ID NO: 8), and cloned into HindIII/EcoRI site in pUC19 to yield pDH144. The fragment is a chimeric composition of nucleotide sequences from the BLZ1-gene (SEQ ID NO: 2) and BLZ2-gene (SEQ ID NO: 4), forming sense and antisense arms, and a 88 bp nucleotide sequence representing intron 4 from the BLZ1 gene (GenBank sequence id. X80068), designed to form RNA hairpin loop after transcription (Figure 4). The 857 bp fragment hpB1/2-nos was released with SacII/EcoRI and substituting the antisenseBLZ1-nos fragment in pDH158 to yield pDH160. The complete insert in pDH160 was released with Sse83871/SgfI digestion and ligated into Sse83871/SgfI site of pKOH250 to generate the binary plant transformation vector pbDH104 (Fig. 5) capable of stable integration of the DNA sequence between the left borders (L) and right borders (R) of the vector into the plant genome.

### EXAMPLE 6

### Genetic transformation of barley with pbDH101 and pDH104

Hordeum vulgaris cv Golden Promise immature seed, about 8 to 14 days post anthesis, is harvested and stored overnight at 4°C in dark. The cold-incubated immature seed is treated in 70% EtOH for 1 min. and then for 10 min. in 0.6% natrium hypochloride, followed by thorough washing (5-8 times) in sterile distilled water an placed on a sterile Petri plate under dissecting microscope in laminar flow hood under sterile conditions. The position of the embryo is located, the end of the seed cut off and a scission made down the side of the seed. The seed is held down with forceps and the middle of the seed pressed down so that the embryo is squeezed out. The embryo is held in place with the forceps, scalpel blade inserted in the groove between scutellum and axis and the axis slowly excised. The scutellum is placed on callus induction media, the cut side up, and inoculated with 25 µl to 40 µl of full-strength Agrobacterium tumefaciens carrying the plant transformation vector for 1 to 5 minutes. After inoculation the scutellum is dragged to the outside of the dish to lower the bacterial load and to reduce the overgrowth during the co-cultivation phase. The infected scutellum is transferred to a new callus induction media plate and the plate incubated at 24°C in dark for 3 days. After 3 days the scutellum is transferred to new callus induction media but with 100 µg/ml timentin for killing off the Agrobacterium and 50 µg/ml hygromycin for selecting transformed cells and incubated for 4 weeks in dark at 24°C, subculturing after 2 weeks. The callus is then transferred to shoot induction media including 2.5 mg/L BAP, 50 µg/ml timentin and 25 µg/ml hygromycin, and incubated in high light for 4 to 10 weeks. Individual regenerating plantlets are transferred to rooting medium (50 µg/ml timentin and 25 µg/ml hygromycin and without hormones). After development to approx. 5 to 7 cm shoots with roots, transgenic plants are moved to soil and grown there under full light.

### EXAMPLE 9

### Accumulation of HoxB4-CBM protein after targetal suppression of BLZ1 and BLZ2

To measure accumulation of the B- and C-hordeins in transgenic plants transformed with either pbDH101 (hoxB4-cbm only) or pbDH104 (BLZ1/2 hpRNA expressed with hoxB4-cbm), Colloidal Coomassie Brilliant Blue G Method may be used as described by Smith (in The Protein Protocols Handbook *ed*. Walker, 2nd ed.,Humana Press, 2002). Soluble proteins from seed extracts are then separated with SDS-PAGE together with standard proteins, the gel subsequently stained for 1 hour with the staining solution (0.04% (w/v) Brilliant Blue G in 3.5% (w/v) perchloric acid), followed by destaining of the gel in distilled water for 4 hours. The intensity of the respective B- and C-hordein bands are analyzed with scanning densitometry and the relative band intensity compared in transgenic plants transformed with pbDH101 or pbDH104. This gives an estimate of the suppression of B- and C-hordein caused by the BLZ1/2 hpRNA. The identity and the level of expression of the HoxB4-CBM in plants transformed with pbDH101 or pbDH104 can be verified and measured by Western blotting of a fragment of the same gel containing a duplicate of samples. The bands may be electroblotted from the gelfragment onto a nitrocellulose membrane using Biorad's Mini transblot Cell but the contours of the gel fragment marked on the filter prior to blotting. After blotting and disassembly of the gel/membrane sandwich, the nitrocellulose is hybridized with polyclonal antisera against CBM, and after multiple washing steps (2*5 min., 2*15 min., 1*5 min.), incubated for 45 min with secondary antibody conjugated to horseradish peroxidase (HRP). After subsequent washing steps, described above, and upon the addition of the substrates 5-bromo-4-chloro-3-indolyl phosphate and nitro blue tetrazolium chloride to the membrane, the HRP color reaction positively identifies the band as HoxB4-CBM and gives and estimation of the effect of BLZ1/2 hpRNA expression on the hoxB4-CBM level.

### REFERENCES

Coles et al. (1999) Plant J. 17:547-556.
Davis et al. (1994): In: Basic Methods in Molecular Biology, Norwalk. Connecticut: Appelton and Lenge: 350-355.
De Blaere et al. (1987) Meth. Enzymol. 143:277.
Fire et al. (1998) Nature 391:806-811.
Klein et al. (1987) Nature 327:70-73.
Maniatis et al. (1982) Molecular Cloning: A Laboratory Manual, Cold Spring Harbor Laboratory Press, Cold Spring Harbor, N.Y.
Muller et al. (1993) Plant J. 4:343-355.
Onate et al. (1999) J. Biol. Chem. 274:9175-9182.
Patel et al. (2000) Molecular Breeding 6:113-123.
Sanford et al. US Patent No. 4,945,050.
Shewmaker et al. US Patent No. 5,759,829.
Shewry et al. (1985) Biochem. Genet. 23:389-402.
Smith et al (2000) Nature 40: 319-320.
Sörensen et al. (1996) Mol. Gen, Genet. 250:750-760.
Takaiwa et al (2003) EP patent application EP 1 327 685 A1
Vicente-Carbajosa et al. (1992) Plant Mol. Biol. 18:453-458.
Vicente-Carbajosa et al. (2998) Plant. J. 13(5):629-640.
Waterhouse et al. (1998) Proc. Natt. Acad. Sci. 95:13959-13964.
Wesley et al. (2001) Plant J. 27:581-590.
Örvar et al. (1997) Plant J. 11:1297-1305.

## Claims

1. A method of enhancing the expression and accumulation of a heterologous polypeptide of interest in plant seeds, said method comprising of:
(a) transforming a plant cell with a DNA sequence comprising a seed-specific promoter operably linked to a DNA sequence encoding one or more transcription regulators (TF), or part(s) thereof, wherein said one or more TF can be a chimeric combination of different TF(s), regulating transcription of one or more endogenous genes encoding seed storage proteins, wherein the transcribed strand of said sequence is capable of suppressing, delaying or otherwise reducing the expression of one or more of said seed storage proteins in said plant cell, and
(b) selecting a seed-specific promoter that has no cis-acting elements recognized by the said transcription regulators in (a), and
(c) transforming the same or another plant cell in (a) with a DNA sequence for a said seed-specific promoter in (b) operably linked to a DNA sequence encoding a heterologous polypeptide of interest;
(d) regenerating a plant from said transformed plant host cell(s), and growing said plant under conditions whereby said DNA sequence(s) encoding one or more TF(s), or part(s) thereof is transcribed, thereby reducing expression of said endogenous mRNA, thus reducing expression of said seed storage proteins, and thus enhancing the expression and accumulation of said heterologous polypeptide of interest.

2. The method of claim 1, wherein the transcribed strand of said sequence encoding one or more transcription regulators (TF) or part(s) thereof is capable of forming a "hairpin" RNA capable of suppressing, delaying or otherwise reducing the expression of one or more of said seed storage proteins in said plant cell.

3. The method of claim 1 or 2, wherein said plant host cell is selected from the group of monocotyledonous plants.

4. The method of claim 3, wherein said plant host cell is selected from the group of monocotyledonous plants containing barley, maize, wheat, oat, and rice.

5. The method of any of claims 1-3 wherein said selected promoter is a Barley D-hordein promoter.

6. The method of claim 1 or 2, wherein the said DNA sequence(s) of step (a) and the said DNA sequence of step (b) are operably linked in one DNA sequence.

7. The method of claim 1 or 2, wherein said DNA sequence(s) of step (a) and said DNA sequences of step (b) are introduced into the same plant cell.

8. The method of claim 1 or 2, wherein the said DNA sequence(s) of step (a) is introduced into the genome of a first plant host cell, and wherein the said DNA sequence of step (b) is introduced into the genome of a second plant host cell.

9. The method of claim 8, wherein a first transgenic plant is regenerated from said first plant host cell and a second transgenic plant is generated from said second plant host cell, and wherein a progeny population of transgenic plants is generated from sexual crossing between said first and second transgenic plant.

10. The method of any of claims 1-9, wherein said one or more seed storage protein is a hordein of barley.

11. The method of any of claims 1-10, wherein said DNA sequence encoding a heterologous polypeptide of interest encodes a protein from the group of prokaryotic or eukaryotic proteins.

12. The method of claim 11 wherein said DNA sequence encoding a heterologous polypeptide of interest encodes a protein from a thermophilic organism.

13. The method of claim 11 or 12 wherein said sequence encodes a carbohydrate binding module (CBM).

14. The method of claim 11, wherein said DNA sequence encoding a prokaryotic or eukaryotic protein is selected from the group consisting of DNA sequences encoding collagens, collagenase, homeobox polypeptides, monoclonal antibodies, secreted antibodies, single chain antibodies, mannose-binding lectin, pepsin, chymotrypsin, trypsin, casein, human growth hormone, human serum albumin, human insulin, cellulases, pectinases, hemicellulases, phytases, hydrolases, peroxidases, fibrinogen, factor IX, Factor XIII, thrombin, protein C, xylanase, Isoamylase, glucoamylase, amylases, lysozyme, beta-glucanase glucocerebrosidase, caseins, lactase, urease, glucose isomerase, invertase, streptavidin, esterases, alkaline phosphatose, protease inhibitors,
papain, kinases, phosphatases, deoxyribonucleases, ribonucleases, phosphilpases, lipases, laccase, spider silk proteins, antifreeze proteins, antimicrobial peptides or defensins, growth factors and cytokinins.

15. The method of claim 14 wherein the said DNA sequence encoding eukaryotic protein is a human homeobox B4 (Hox84) gene encoding HoxB4 protein.

16. The method of claim 15 wherein the said HoxB4 protein has at least 70% sequence Identity to the amino acid sequence of SEQ ID NO: 1.

17. The method of any of claims 1-16, wherein said DNA sequence encoding one or more TF is a chimeric DNA sequence comprised of regions of two or more DNA sequences encoding TF-s, or parts thereof.

18. The method of any of claims 1-17, wherein said DNA sequence encoding a TF or part thereof comprises a region encoding a TF or part thereof from the group of bZIP proteins.

19. The method of claim 18, wherein said DNA sequence encoding a BZIP protein comprises a DNA sequence selected from
a) a DNA sequence encoding barley BLZ1 protein or a part thereof:
b) a DNA sequence encoding barley BLZ2 protein or a part thereof;
c) a combination of a) and b).

20. The method of claim 19, wherein said DNA sequence is selected from SEQ ID NO: 2, SEQ ID NO: 3, SEQ ID NO: 4, SEQ ID NO: 5, and any part or combination thereof.

21. The method of claim 1 wherein said DNA sequence encoding TF is capable of expressing a hairpin RNA (hpRNA) in said plant cell.

22. The method of claim 21 wherein said DNA sequence encoding TF comprises a region or part thereof encoding a TF from the group of bZIP proteins.

23. The method of claim 22 wherein said bZIP protein is selected from BLZ1, BLZ2 and a chimeric combination of BLZ1 and BLZ2.

24. The method of claim 23 wherein said DNA sequence comprises a sequence selected from SEQ ID NO: 2, SEQ ID NO: 4, SEQ ID NO: 6, or SEQ ID NO: 7, SEQ ID NO: 8 and any part or combination thereof.

25. A transgenic Barley plant being transformed with:
a) a DNA sequence comprising a seed-specific promoter operably linked to a DNA sequence encoding one or more transcription regulators (TF), or part(s) thereof, including a chimeric combination of different TF(s), regulating transcription of one or more endogenous genes encoding seed storage proteins,
b) a seed-specific promoter that has no cis-acting elements recognized by the said transcription regulators operably linked to a DNA sequence encoding a heterologous protein of interest,
wherein said plant expresses in its seeds said heterologous protein and expresses substantially less seed storage proteins than a corresponding non-recombinant plant.

## Patentansprüche

1. Verfahren zur Steigerung der Expression und Akkumulation eines heterologen Polypeptids von Interesse in Pflanzensamen, wobei das Verfahren Folgendes umfasst:
(a) das Transformieren einer Pflanzenzelle mit einer DNA-Sequenz, die einen samenspezifischen Promotor umfasst, der wirkungsmäßig mit einer DNA-Sequenz verbunden ist, die einen oder mehrere Transkriptionsregulatoren (TF) oder (einen) Teil(e) davon codiert, wobei der eine oder die mehreren TF eine chimäre Kombination unterschiedlicher TFs sein kann/können, welche die Transkription von einem oder mehreren endogenen Genen regulieren, welche Samenspeicherproteine codieren, wobei der transkribierte Strang der Sequenz in der Lage ist, die Expression von einem oder mehreren der Samenspeicherproteine in der Pflanzenzelle zu unterdrücken, zu verzögern oder anderweitig zu reduzieren, und
(b) das Auswählen eines samenspezifischen Promotors, der keine cis-agierenden Elemente aufweist, welche durch die Transkriptionsregulatoren unter (a) erkannt werden, und
(c) das Transformieren der gleichen oder einer anderen Pflanzenzelle unter (a) mit einer DNA-Sequenz für den samenspezifischen Promotor unter (b), der wirkungsmäßig mit einer DNA-Sequenz verbunden ist, die ein heterologes Polypeptid von Interesse codiert;
(d) das Regenerieren einer Pflanze aus der/den transformierten Pflanzenwirtszelle(n) und das Züchten der Pflanze unter Bedingungen, unter welchen die DNA-Sequenz(en), die den einen oder die mehreren TF(s) oder (einen) Teil(e) davon codiert/codieren, transkribiert wird/werden, wodurch die Expression der endogenen mRNA reduziert wird, wodurch die Expression der Samenspeicherproteine reduziert wird und wodurch die Expression und Akkumulation des heterologen Polypeptids von Interesse gesteigert wird.

2. Verfahren nach Anspruch 1, wobei der transkribierte Strang der Sequenz, welche einen oder mehrere Transkriptionsregulatoren (TF) oder (einen) Teil(e) davon codiert, in der Lage ist, eine "Haarnadel"-RNA zu bilden, die in der Lage ist, die Expression von einem oder mehreren der Samenspeicherproteine der Pflanzenzelle zu unterdrücken, zu verzögern oder anderweitig zu reduzieren.

3. Verfahren nach Anspruch 1 oder 2, wobei die Pflanzenwirtszelle ausgewählt ist aus der Gruppe der monokotylen Pflanzen.

4. Verfahren nach Anspruch 3, wobei die Pflanzenwirtszelle ausgewählt ist aus der Gruppe der monokotylen Pflanzen, einschließlich Gerste, Mais, Weizen, Hafer und Reis.

5. Verfahren nach einem der Ansprüche 1-3, wobei der ausgewählte Promotor ein Gersten-D-Hordein-Promotor ist.

6. Verfahren nach Anspruch 1 oder 2, wobei die DNA-Sequenz(en) aus Schritt (a) und die DNA-Sequenz aus Schritt (b) wirkungsmäßig in einer DNA-Sequenz verbunden sind.

7. Verfahren nach Anspruch 1 oder 2, wobei die DNA-Sequenz(en) aus Schritt (a) und die DNA-Sequenzen aus Schritt (b) in die gleiche Pflanzenzelle eingeführt sind.

8. Verfahren nach Anspruch 1 oder 2, wobei die DNA-Sequenz(en) aus Schritt (a) in das Genom einer ersten Pflanzenwirtszelle eingeführt ist/sind und wobei die DNA-Sequenz aus Schritt (b) in das Genom einer zweiten Pflanzenwirtszelle eingeführt ist.

9. Verfahren nach Anspruch 8, wobei eine erste transgene Pflanze aus der ersten Pflanzenwirtszelle regeneriert wird und eine zweite transgene Pflanze aus der zweiten Pflanzenwirtszelle erzeugt wird, und wobei eine Nachkommenpopulation transgener Pflanzen aus der geschlechtlichen Kreuzung zwischen der ersten und zweiten transgenen Pflanze erzeugt wird.

10. Verfahren nach einem der Ansprüche 1-9, wobei das eine oder die mehreren Samenspeicherprotein(e) ein Hordein der Gerste ist.

11. Verfahren nach einem der Ansprüche 1-10, wobei die DNA-Sequenz, die ein heterologes Polypeptid von Interesse codiert, ein Protein aus der Gruppe der prokaryotischen oder eukaryotischen Proteine codiert.

12. Verfahren nach Anspruch 11, wobei die DNA-Sequenz, die ein heterologes Polypeptid von Interesse codiert, ein Protein aus einem thermophilen Organismus codiert.

13. Verfahren nach Anspruch 11 oder 12, wobei die Sequenz ein Kohlenhydratbindungsmodul (CBM) codiert.

14. Verfahren nach Anspruch 11, wobei die DNA-Sequenz, die ein prokaryotisches oder eukaryotisches Protein codiert, ausgewählt ist aus der Gruppe bestehend aus DNA-Sequenzen, die Kollagene, Kollagenase, Homöobox-Polypeptide, monoklonale Antikörper, sekretierte Antikörper, Einzelketten-Antikörper, Mannose-bindendes Lektin, Pepsin, Chymotrypsin, Trypsin, Casein, humanes Wachstumshormon, humanes Serumalbumin, humanes Insulin, Cellulasen, Pektinasen, Hemicellulasen, Phytasen, Hydrolasen, Peroxidasen, Fibrinogen, Faktor IX, Faktor XIII, Thrombin, Protein C, Xylanase, Isoamylase, Glucoamylase, Amylasen, Lysozym, Beta-Glucanase, Glukozerebrosidase, Caseine, Laktase, Urease, Glukoseisomerase, Invertase, Streptavidin, Esterasen, alkalische Phosphatase, Protease-Inhibitoren, Papain, Kinasen, Phosphatasen, Deoxyribonukleasen, Ribonukleasen, Phospholipasen, Lipasen, Laccase, Spinnenseidenproteine, Gefrierschutzproteine, antimikrobielle Peptide oder Defensine, Wachstumsfaktoren und Zytokine codieren.

15. Verfahren nach Anspruch 14, wobei die DNA-Sequenz, die eukaryotisches Protein codiert, ein humanes Homöobox-B4 (HoxB4) - Gen ist, welches HoxB4-Protein codiert.

16. Verfahren nach Anspruch 15, wobei das HoxB4-Protein mindestens 70 % Sequenzidentität mit der Aminosäuresequenz von SEQ ID NR.: 1 aufweist.

17. Verfahren nach einem der Ansprüche 1-16, wobei die DNA-Sequenz, die einen oder mehrere TF codiert, eine chimäre DNA-Sequenz ist, die aus Regionen von zwei oder mehr DNA-Sequenzen besteht, welche TFs oder Teile davon codieren.

18. Verfahren nach einem der Ansprüche 1-17, wobei die DNA-Sequenz, die einen TF oder einen Teil davon codiert, eine Region, die einen TF oder einen Teil davon codiert, aus der Gruppe der bZIP-Proteine umfasst.

19. Verfahren nach Anspruch 18, wobei die DNA-Sequenz, die ein bZIP-Protein codiert, eine DNA-Sequenz ausgewählt aus
a) einer DNA-Sequenz, die Gersten-BLZ1-Protein oder einen Teil davon codiert,
b) einer DNA-Sequenz, die Gersten-BLZ2-Protein oder einen Teile davon codiert,
c) einer Kombination aus a) und b)
umfasst.

20. Verfahren nach Anspruch 19, wobei die DNA-Sequenz ausgewählt ist aus SEQ ID NR.: 2, SEQ ID NR.: 3, SEQ ID NR.: 4, SEQ ID NR.: 5 und jedem Teil oder jeder Kombination daraus.

21. Verfahren nach Anspruch 1, wobei die DNA-Sequenz, die TF codiert, in der Lage ist, eine Haarnadel-RNA (hpRNA) in der Pflanzenzelle zu exprimieren.

22. Verfahren nach Anspruch 21, wobei die DNA-Sequenz, die TF codiert, eine Region oder einen Teil davon umfasst, welche/r einen TF aus der Gruppe der bZIP-Proteine codiert.

23. Verfahren nach Anspruch 22, wobei das bZIP-Protein ausgewählt ist aus BLZ1, BLZ2 und einer chimären Kombination aus BLZ1 und BLZ2.

24. Verfahren nach Anspruch 23, wobei die DNA-Sequenz eine Sequenz ausgewählt aus SEQ ID NR.: 2, SEQ ID NR.: 4, SEQ ID NR.: 6 oder SEQ ID NR.: 7, SEQ ID NR.: 8 und jedem Teil oder jeder Kombination daraus umfasst.

25. Transgene Gerstenpflanze transformiert mit:
a) einer DNA-Sequenz, die einen samenspezifischen Promotor umfasst, der wirkungsmäßig mit einer DNA-Sequenz verbunden ist, die einen oder mehrere Transkriptionsregulatoren (TF) oder (einen) Teil(e) davon codiert, einschließlich einer chimären Kombination unterschiedlicher TFs, welche die Transkription eines oder mehrerer endogener Gene regulieren, die wiederum Samenspeicherproteine codieren,
b) einem samenspezifischen Promotor, der keine cis-agierenden Elemente aufweist, die durch die Transkriptionsregulatoren erkannt werden, welche wirkungsmäßig mit einer DNA-Sequenz verbunden sind, die ein heterologes Protein von Interesse codieren,
wobei die Pflanzen in ihren Samen das heterologe Protein exprimiert und wesentlich weniger Samenspeicherprotein als eine entsprechende nichtrekombinante Pflanze exprimiert.

## Revendications

1. Procédé d'amélioration de l'expression et de l'accumulation d'un polypeptide hétérologue d'intérêt dans les graines de plantes, ledit procédé consistant à :
(a) transformer une cellule de plante avec une séquence d'ADN comprenant un promoteur spécifique des graines opérationnellement lié à une séquence d'ADN codant pour un ou plusieurs régulateur(s) de la transcription (TF), ou une/des partie(s) de celui-ci/ceux-ci, dans lequel ledit/lesdits TF peut/peuvent être une combinaison chimérique de différents TF, en régulant la transcription d'un ou plusieurs gène(s) endogène(s) codant pour les protéines de stockage des graines, dans lequel le brin transcrit de ladite séquence est capable de supprimer, retarder ou encore réduire l'expression d'une ou plusieurs desdites protéines de stockage des graines dans ladite cellule de plante, et
(b) sélectionner un promoteur spécifique des graines qui ne possède pas d'éléments cis-régulateurs reconnus par lesdits régulateurs de la transcription dans (a), et
(c) transformer une cellule de plante identique ou différente dans (a) avec une séquence d'ADN pour un dit promoteur spécifique des graines dans (b) opérationnellement lié à une séquence d'ADN codant pour un polypeptide hétérologue d'intérêt ;
(d) régénérer une plante à partir de ladite/desdites cellule(s) hôte(s) transformée(s) de plantes et cultiver ladite plante sous les conditions dans lesquelles ladite/lesdites séquence(s) d'ADN codant pour un ou plusieurs TF, ou une/des partie(s) de celui-ci/ceux-ci, est/sont transcrite(s), en réduisant ainsi l'expression dudit ARNm endogène, en réduisant ainsi l'expression desdites protéines de stockage des graines, et en améliorant ainsi l'expression et l'accumulation dudit polypeptide hétérologue d'intérêt.

2. Procédé de la revendication 1, dans lequel le brin transcrit de ladite séquence codant pour un ou plusieurs régulateur(s) de la transcription (TF) ou une/des partie(s) de celui-ci/ceux-ci est capable de former un ARN « en épingle à cheveux » capable de supprimer, de retarder ou encore de réduire l'expression d'une ou plusieurs desdites protéines de stockage des graines dans ladite cellule de plante.

3. Procédé de la revendication 1 ou 2, dans lequel ladite cellule de plante hôte est sélectionnée dans le groupe constitué par les plantes monocotylédones.

4. Procédé de la revendication 3, dans lequel ladite cellule de plante hôte est sélectionnée dans le groupe constitué par les plantes monocotylédones contenant l'orge, le maïs, le blé, l'avoine et le riz.

5. Procédé de l'une quelconque des revendications 1 à 3, dans lequel ledit promoteur sélectionné est un promoteur de la D-hordéine d'orge.

6. Procédé de la revendication 1 ou 2, dans lequel ladite/lesdites séquence(s) d'ADN de l'étape (a) et ladite séquence d'ADN de l'étape (b) sont opérationnellement liées dans une séquence d'ADN.

7. Procédé de la revendication 1 ou 2, dans lequel ladite/lesdites séquence(s) d'ADN de l'étape (a) et ladite séquence d'ADN de l'étape (b) sont introduites dans la même cellule de plante.

8. Procédé de la revendication 1 ou 2, dans lequel ladite/lesdites séquence(s) d'ADN de l'étape (a) est introduite dans le génome d'une première cellule de plante hôte, et dans lequel ladite séquence d'ADN de l'étape (b) est introduite dans le génome d'une deuxième cellule de plante hôte.

9. Procédé de la revendication 8, dans lequel une première plante transgénique est régénérée à partir de ladite première cellule de plante hôte et une deuxième plante transgénique est générée à partir de ladite deuxième cellule de plante hôte, et dans lequel une population de progénitures de plantes transgéniques est générée par croisement sexuel entre lesdites première et deuxième plantes transgéniques.

10. Procédé de l'une quelconque des revendications 1 à 9, dans lequel ladite ou lesdites protéine(s) de stockage des graines est/sont une hordéine d'orge.

11. Procédé de l'une quelconque des revendications 1 à 10, dans lequel ladite séquence d'ADN codant pour un polypeptide hétérologue d'intérêt code pour une protéine du groupe des protéines procaryotes ou eucaryotes.

12. Procédé de la revendication 11, dans lequel ladite séquence d'ADN codant pour un polypeptide hétérologue d'intérêt code pour une protéine d'un organisme thermophile.

13. Procédé de la revendication 11 ou 12, dans lequel ladite séquence code pour une molécule de liaison glucidique (CBM).

14. Procédé de la revendication 11, dans lequel ladite séquence d'ADN codant pour une protéine procaryote ou eucaryote est sélectionnée dans le groupe constitué par les séquences d'ADN codant pour les collagènes, la collagénase, les polypeptides à homéoboîte, les anticorps monoclonaux, les anticorps sécrétés, les anticorps monocaténaires, la lectine de liaison au mannose, la pepsine, la chymotrypsine, la trypsine, la caséine, l'hormone de croissance humaine, la sérumalbumine humaine, l'insuline humaine, les cellulases, les pectinases, les hémicellulases, les phytases, les hydrolases, les peroxydases, le fibrinogène, le facteur IX, le facteur XIII, la thrombine, la protéine C, la xylanase, l'isoamylase, la glucoamylase, les amylases, le lysozyme, la bêta-glucanase, la glucocérébrosidase, les caséines, la lactase, l'uréase, la glucose isomérase, l'invertase, la streptavidine, les estérases, l'alcaline phosphatase, les inhibiteurs de la protéase, la papaïne, les kinases, les phosphatases, les désoxyribonucléases, les ribonucléases, les phospholipases, les lipases, la laccase, les protéines de soie d'araignée, les protéines antigel, les peptides antimicrobiens ou les défensines, les facteurs de croissance et les cytokinines.

15. Procédé de la revendication 14, dans lequel ladite séquence d'ADN codant pour la protéine eucaryote est un gène humain à homéoboîte B4 (HoxB4) codant pour la protéine HoxB4.

16. Procédé de la revendication 15, dans lequel ladite protéine HoxB4 présente une identité de séquences d'au moins 70 % avec la séquence d'acides aminés de SEQ ID n°: 1.

17. Procédé de l'une quelconque des revendications 1 à 16, dans lequel ladite séquence d'ADN codant pour un ou plusieurs TF est une séquence d'ADN chimérique constituée des régions de deux séquences d'ADN ou plus codant pour les TF, ou des parties de ceux-ci.

18. Procédé de l'une quelconque des revendications 1 à 17, dans lequel ladite séquence d'ADN codant pour un TF ou une partie de celui-ci comprend une région codant pour un TF ou une partie de celui-ci du groupe des protéines bZIP.

19. Procédé de la revendication 18, dans lequel ladite séquence d'ADN codant pour une protéine bZIP comprend une séquence d'ADN sélectionnée parmi :
a) une séquence d'ADN codant pour la protéine BLZ1 de l'orge ou une partie de celle-ci ;
b) une séquence d'ADN codant pour la protéine BLZ2 de l'orge ou une partie de celle-ci ;
c) une combinaison de a) et b).

20. Procédé de la revendication 19, dans lequel ladite séquence d'ADN est sélectionnée parmi SEQ ID n° : 2, SEQ ID n° : 3, SEQ ID n° : 4, SEQ ID n° : 5 et une quelconque partie ou combinaison de celles-ci.

21. Procédé de la revendication 1, dans lequel ladite séquence d'ADN codant pour le TF est capable d'exprimer un ARN en épingle à cheveux (ARNhp) dans ladite cellule de plante.

22. Procédé de la revendication 21, dans lequel ladite séquence d'ADN codant pour le TF comprend une région ou une partie de celle-ci codant pour un TF du groupe des protéines bZIP.

23. Procédé de la revendication 22, dans lequel ladite protéine bZIP est sélectionnée parmi BLZ1, BLZ2 et une combinaison chimérique de BLZ1 et BLZ2.

24. Procédé de la revendication 23, dans lequel ladite séquence d'ADN comprend une séquence sélectionnée parmi SEQ ID n° : 2, SEQ ID n° : 4, SEQ ID n° : 6, SEQ ID n° : 7 ou SEQ ID n° : 8 et une quelconque partie ou combinaison de celles-ci.

25. Plante de type orge transgénique transformée avec :
a) une séquence d'ADN comprenant un promoteur spécifique des graines opérationnellement lié à une séquence d'ADN codant pour un ou plusieurs régulateur(s) de la transcription (TF), ou une/des partie(s) de celui-ci/ceux-ci, notamment une combinaison chimérique de différents TF, en régulant la transcription d'un ou plusieurs gène(s) endogène(s) codant pour les protéines de stockage des graines,
b) un promoteur spécifique des graines qui ne possède pas d'éléments cis-régulateurs reconnus par lesdits régulateurs de la transcription opérationnellement liés à une séquence d'ADN codant pour une protéine hétérologue d'intérêt,
dans laquelle ladite plante exprime dans ses graines ladite protéine hétérologue d'intérêt et exprime sensiblement moins de protéines de stockage des graines qu'une plante non recombinante correspondante.
